# EUROPÄISCHE PATENTSCHRIFT

(11) **EP 1 044 030 B1**
(45) Veröffentlichungstag und Bekanntmachung des Hinweises auf die Patenterteilung: **29.10.2003**
(21) Anmeldenummer: 97954758.5
(22) Anmeldetag: 24.12.1997
(51) Int. Cl.: A61M 16/04

(54) **TRACHEOSTOMAPLATZHALTER**
TRACHEOSTOMA PLACEHOLDER
DISPOSITIF D'ACCES POUR TRACHEOTOMIE

(43) Veröffentlichungstag der Anmeldung: 18.10.2000
(73) Patentinhaber: PRIMED HALBERSTADT MEDIZINTECHNIK GMBH, 38820 Halberstadt (DE)
(72) Erfinder: MÜLLER, Andreas, D-07743 Jena (DE); NEUBAUER, Norbert, D-38820 Halberstadt (DE)
(74) Vertreter: Bock, Gerhard
(86) Internationale Anmeldenummer: EP9707311
(87) Internationale Veröffentlichungsnummer: WO99033507

(56) Entgegenhaltungen:
- DE-A- 19 636 050
- DE-U- 8 514 859
- US-A- 3 973 569
- US-A- 4 269 184
- US-A- 5 683 458

## Beschreibung

Die Erfindung betrifft einen Tracheostomaplatzhalter zum Abdichten einer vorübergehenden Tracheaeröffnung und Bildung einer sicheren Zugangsmöglichkeit zur Luftröhre gemäß dem Oberbegriff des Anspruchs 1.

Derartige Tracheostomaplatzhalter finden bevorzugt in folgenden Anwendungsgebieten Einsatz:
- Entwöhnung von Luftröhrenkanülen und Beatmungstuben nach Langzeitbeatmung oder nach einer Kehlkopf- oder Luftröhrenchirurgie,
- Gewährleistung eines sicheren Zugangs zur Luftröhre bei Gefahr einer Atemwegsverlegung oder -blutung nach chirurgischen Eingriffen an Pharynx/Larynx, Tumoren,
- Sekretabsaugmöglichkeit aus tieferen Atemwegen bei schweren pulmonalen oder neurologischen Erkrankungen,
- bei wiederkehrenden Atemwegsverlegungen im Bereich des Schlundes oder des Kehlkopfes, wie im Falle von obstruktiven Schlafapnoe, gestörter Glottismotorik oder bei bestrahlungsbedingter Verschwellung.

Ebenso kann die erfindungsgemäße Lösung in Sonderanwendungen, wie bei Einatemhindernissen, z.B. bei doppelseitiger Stimmbandlähmung, Einsatz finden.

Es sind verschiedene Tracheostomaplatzhalter bekannt. So bspw. ein sogenannter "Barton-MayoTracheostoma Button" (bess-Firmenprospekt 1996), der aus einem einstöckigem Teil bestehend aus einer Ventilaufnahme, einem Tracheostomaschenkel und einer in die Stoma eindrückbaren knopfartigem, konzentrischem Abschluß besteht. Dieser Platzhalter hat den Nachteil, daß er den jeweiligen anatomischen Besonderheiten nur durch eine spezielle Neuanfertigung anpaßbar wäre, über keine besonders gute Dichtwirkung verfügt und vor allem beim Patienten durch eine relativ scharfkantige Anlage an der Halshaut zu Schwellungen und Schmerzen führt. Bei anderen bekannten Abstandshaltern (z.B. US-PS 4,269,184) wird versucht, die trachealseitige Anlage des Abstandshalters dadurch zu verbessern, daß an dem Tracheostomaschenkel diametral gegenüberstehend zwei gleichlange, lappenartige Anlagen vorgesehen sind. Auch diese Lappen stellen keine sichere Anlage an die Trachea dar und neigen sehr leicht zur Dislokation. Allen bekannten Tracheostomaplatzhaltern zum Abdichten einer vorübergehenden Tracheaeröffnung und Bildung einer sicheren Zugangsmöglichkeit zur Luftröhre ist gemein, daß die Trageeigenschaften beim Patienten als unangenehm empfunden werden.

Weiterhin sind für andere Einsatzgebiete, nämlich für die Ventilabdichtung des Luftröhrenstumpfes nach einer Kehlkopftotalentfernung, Tracheostoma-Verschlüsse aus DE-U-85 14 859 und US-PS 5,683,458 bekannt. Um eine sprachliche Kommunikation zu erreichen, wird diesen Patienten ein sogenanntes Stimmventil zwischen Luftröhre und Speiseröhre eingesetzt. Dieses ermöglicht bei Abdichtung der äußeren Luftröhrenstumpföffnung am Hals beim Ausatmen das Einpressen von Luft in die Speiseröhre und damit in den Mundrachen. dort wird eine Ersatzstimme ausgebildet und mit der Zunge und den Lippen ausgeformt. Normalerweise halten sich die Patienten diese Öffnung mit dem Finger zu. Da dies hygienische Probleme verursacht, wurden eine Reihe von Ventilen entwickelt, die auch wie die vorstehend genannten, eine kurzzeitige Blockierung der Ausatmung zum Einpressen von Luft in das Stimmventil ermöglichen. Das Ziel solcher Stents ist also die Aufnahme eines Stimmventils und die Verankerung in der Luftröhrenstumpföffnung. Aufgrund der Kehlkopfentfernung atmet der Patient über die Luftröhrenstumpföffnung und damit durch den Stent. Eine Zuführmöglichkeit bspw. einer Atemkanüle oder eines Beatmungstubus ist bei diesen Lösungen nicht möglich.

Der Erfindung liegt die Aufgabe zugrunde, einen Tracheostomaplatzhalter zum Abdichten einer vorübergehenden Tracheaeröffnung und Bildung einer sicheren Zugangsmöglichkeit zur Luftröhre anzugeben, der die Nachteile des Standes der Technik behebt, einen sicheren und weitestgehend selbstdichtenden Sitz in der Luftröhrenöffnung gewährleistet und darüber hinaus weitere Einsatzgebiete erschließt.

Die Aufgabe wird durch die Merkmale des Patentanspruchs 1 gelöst. Vorteilhafte weitere Ausbildungen sind durch die nachgeordneten Ansprüche erfaßt.

Die Erfindung soll nachstehend anhand eines Ausführungsbeispiels und schematischer Zeichnungen näher erläutert werden. Es zeigen:
- Fig. 1: eine Ausbildung eines Tracheostomaplatzhalters in seitlicher Ansicht,
- Fig. 2: die Ausbildung nach Figur 1 in Draufsicht,
- Fig. 3: ein zum Einsatz gelangendes Schild,
- Fig. 3a: ein weiteres modifiziertes Schild,
- Fig. 4: eine besondere Ausbildung eines Verschlußmittels in teilweise geschnittener Darstellung und
- Fig. 5: eine bevorzugte Trachealschenkelausbildung

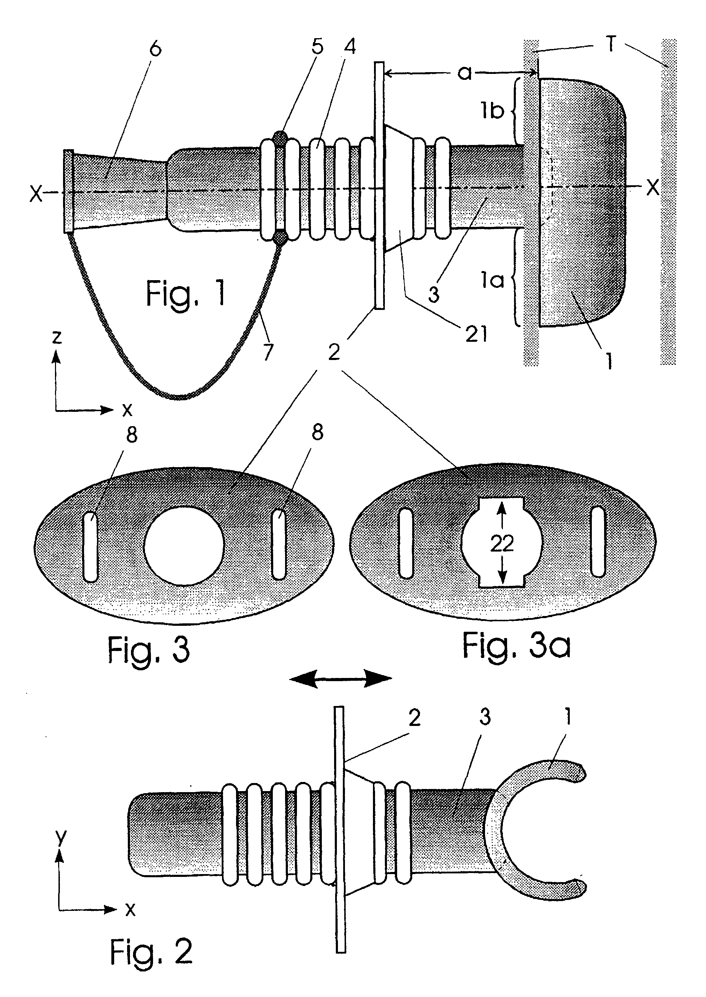

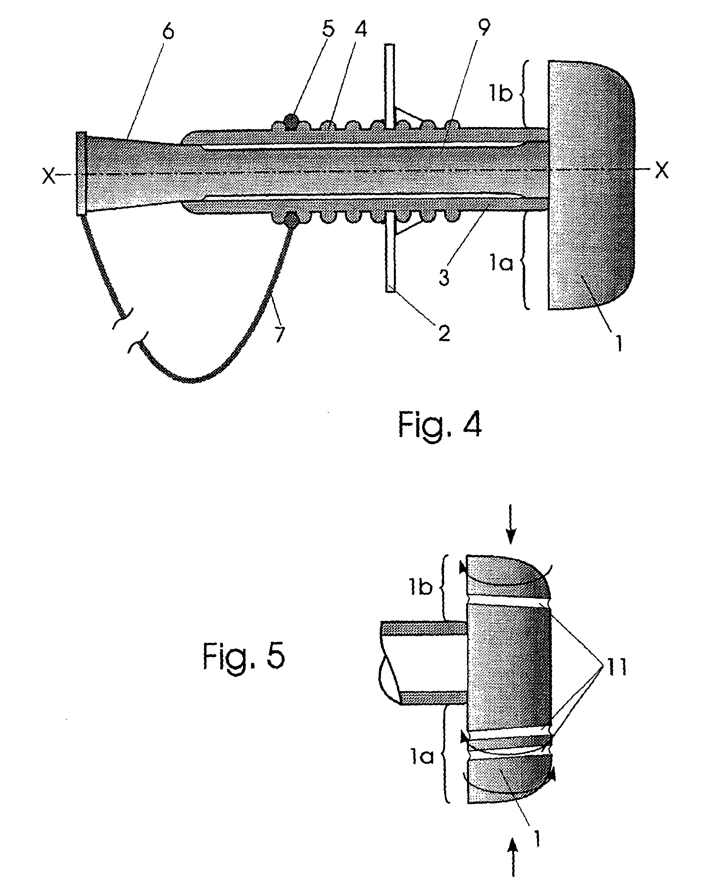

In Figur 1 ist ein Tracheostomaplatzhalter nach der Erfindung in seitlicher Ansicht dargestellt, der aus einem Tracheostomaschenkel 3 und einem Trachealschenkel 1 besteht; beide Teile bilden eine einstückige Einheit. Der Trachealschenkel 1 ist gewölbt ausgeführt und ist im Beispiel senkrecht zur Tracheostomaschenkelachse X-X amittig derart angebunden, daß er unterschiedlich lange Überhangbereiche 1a, 1b aufweist, wobei das Verhältnis der maximalen Längen der Überhangbereiche 1a : 1b vorzugsweise in der Größenordnung von 1,5 festgelegt ist. Insbesondere ist der Trachealschenkel 1 derart ausgebildet, daß er atraumatisch und ellipsenförmig in Richtung der großen Halbachse gewölbt, der Trachea (T) angepaßt ist. Diese Ausbildung ist in Figur 2, die eine Draufsicht auf Figur 1 darstellt, nochmals verdeutlicht. Der Trachealschenkel 1 wird mit dem längeren Teil 1a nach unten in die Trachea T eingeführt und danach vorsichtig der kürzere Teil 1b mittels einer anatomischen Pinzette durch das Tracheostoma in die Trachea T nach oben eingebracht. Dem Tracheostomaschenkel 3 sind weiterhin Rastmittel 4 zugeordnet, die in Form von in den Tracheostomaschenkel 3 eingebrachten Nuten oder in Form von auf den Tracheostomaschenkel 3 aufgebrachten Wulsten ausgebildet sind. Die Nuten bzw. Wulste weisen dabei bevorzugt unterbrochene Bereiche auf, also keine kreisringförmig geschlossene Ausbildung darstellen. Die Elemente Trachealschenkel 1, Tracheostomaschenkel 3 und Rastmittel 4 sind als einstückiges Spritzteil aus einem elastischen medizinischen Werkstoff, bevorzugt einem Silikon, gefertigt. Die Rastmittel 4 dienen der Aufnahme und Fixierung eines Schildes 2, welches durch Verschieben in Richtung eines in Figur 2 dargestellten Doppelpfeiles einen variabel, den jeweiligen anatomischen Gegebenheiten anpaßbaren Abstand a zur Anlagefläche des Trachealschenkels 1 an die Trachea T und damit einen sicheren und dichtenden Abschluß gewährleistet. Eine Ausbildung des Schildes 2 ist in Figur 3 dargestellt. Dem Schild 2 sind weiterhin ein Dichtungskegel 21, bestehend aus einem weichen Kunststoffmaterial (vgl. Fig. 1), und Befestigungselemente 8 zur Aufnahme eines Halte- oder Klebebandes zugeordnet. Weiterhin kann dem Schild 2, zumindest in seinen Außenberandungsbereichen eine anatomisch an den Hals angepaßte Wölbung gegeben sein. Eine bevorzugte Ausbildung des Schildes 2 ist in Fig. 3a dargestellt. Die mittige Schildausnehmung weist dabei Ausnehmungen 22 auf, die im Beispiel rechteckförmig gestaltet sind. Der kreisförmige Bereich der mittigen Schildausnehmung entspricht in seinem Durchmesser dem Außendurchmesser des Tracheostomaschenkels 3, wohingegen die Ausnehmungen 22 mit entsprechend ausgebildeten, umfangsmäßig unterbrochenen, wulstförmigen Rastmitteln 4 bspw. durch Verdrehen um 90° in Eingriff bringbar sind.
In weiterer Ausbildung der Erfindung ist der Tracheostomaschenkel 3 an dem, dem Trachealschenkel 1 abseitigen Ende mit einer weiteren Baugruppe versehbar, welche insbesondere aus einem Verschlußmittel, bestehend aus einem Stopfen 6, der über eine Verlustsicherung 7 mit einem Ring 5 verbunden ist, gebildet ist. Der Stopfen 6, die Verlustsicherung 7 und der Ring 5 sind ihrerseits als einstückiges Spritzteil ausgebildet, wobei der Ring 5 in die Rastmittel 4 des Tracheostomaschenkels 3 eingreifend angeordnet ist. Vorzugsweise weist der Stopfen 6 eine einstückig angeformte, in den Tracheostomaschenkel 3 eingreifende und zumindest im Bereich der Austrittsöffnung im Fußanbindungsgebiet des Trachealschenkels 1 dichtend anliegende stempelartige Verlängerung 9 auf, siehe Figur 4.
Die besondere Ausbildung des aufgezeigten Verschlußmittels ermöglicht ferner thren Austausch und die Anbindung weiterer Baugruppen, wie eines Universalkonnektors für eine assistierte Beatmung über genormte Anschlüsse. Mit dem geschaffenen Tracheostomaplatzhalter liegt somit eine Vorrichtung für multivalente Einsatzgebiete vor, die den anatomischen Besonderheiten des Patienten Rechnung trägt und einen sicheren und komfortablen Sitz gewährleistet, In bevorzugter weiterer Ausbildung sind im Trachelschenkel 1 Abschnitte 11 vorgesehen, die insbesondere als ausgedünnte Bereiche ausgebildet sind. Dadurch wird der Trachelschenkel 1 in einem gewissen Maße in Richtung der in Figur 5 dargestellten Pfeile stauchbar und die durch die Abschnitte 11 geschaffenen einzelnen Trachealschenkelbereiche sind in gewissem Maße gegeneinander verdrehbar, wodurch Schleimhautschäden in der Luftröhre weitestgehend vermieden werden.
Je nach anatomischen Gegebenheiten liegt es im Rahmen der Erfindung, den Trachealschenkel 1 in einem gewissen Winkel zum Tracheostomaschenkel 3 anzuordnen, wobei in diesem Fall den Wulsten bzw. Ausnehmungen der Rastmittel und damit der Stellung des Schildes eine analoge parallel verdrehte Anordnung gegeben ist.

### Bezugszeichenliste

- 1 -: Trachealschenkel
- 11 -: ausgedünnte Trachealschenkelabschnitte
- 1a -: längerer Überhangbereich
- 1b -: kürzerer Überhangbereich
- 2 -: Schild
- 21 -: Dichtungskegel
- 22 -: Ausnehmungen in der mittigen Schildausnehmung
- 3 -: Tracheostomaschenkel
- 4 -: Rastmittel
- 5 -: Ring
- 6 -: Stopfen
- 7 -: Verlustsicherung
- 8 -: Befestigungselemente
- 9 -: stempelartige Verlängerung
- a -: Abstand
- T -: Trachea
- X-X -: Tracheostomaschenkelachse

## Patentansprüche

1. Tracheostomaplatzhalter zum Abdichten einer vorübergehenden Tracheaeröffnung und Bildung einer sicheren Zugangsmöglichkeit zur Luftröhre, bestehend aus einem rohrförmigen Tracheostomaschenkel (3), der einseitig in einen einstückig angeformten und in einer ersten Dimension (y) gewölbt ausgebildeten Trachealschenkel (1) ausläuft, wobei der Trachealschenkel (1) in einer zur ersten Dimension senkrecht verlaufenden Dimension (z) amittig zur Tracheostomaschenkelachse (X-X) derart angebunden ist, daß er unterschiedlich lange Überhangbereiche (1a, 1b) aufweist, wobei der Tracheostomaschenkel (3) an dem, dem Trachealschenkel (1) abseitigen Ende mit einem Verschlußmittel versehbar ist, **dadurch gekennzeichnet, daß** die Überhangbereiche (1a, 1b) mit mehreren ausgedünnten Trachealschenkelabschnitten (11) versehen sind, und dem Tracheostomaschenkel (3) Rastmittel (4) zugeordnet sind, die die Aufnahme und Fixierung eines Schildes (2) in einem variabel anpaßbaren Abstand (a) zur Anlagefläche des Trachealschenkels (1) an die Trachea (T) gewährleisten.

2. Tracheostomaplatzhalter nach Anspruch 1, **dadurch gekennzeichnet, daß** der Trachealschenkel (1) atraumatisch und ellipsenförmig in Richtung der großen Halbachse, der Trachea (T) angepaßt, gewölbt ausgebildet ist.

3. Tracheostomaplatzhalter nach Anspruch 1, **dadurch gekennzeichnet, daß** der längere Überhangbereich (1a) in der Größenordnung von einem 1,5fachen der Länge des kürzeren Überhangbereiches (1b) festgelegt ist.

4. Tracheostomaplatzhalter nach Anspruch 1, **dadurch gekennzeichnet, daß** die Elemente Trachealschenkel (1), Tracheostomaschenkel (3) und Rastmittel (4) als einstückiges Spritzteil aus einem elastischen medizinischen Werkstoff gefertigt sind.

5. Tracheostomaplatzhalter nach Anspruch 4, **dadurch gekennzeichnet, daß** die Elemente Trachealschenkel (1), Tracheostomaschenkel (3) und Rastmittel (4) aus einem Silikon gefertigt sind.

6. Tracheostomaplatzhalter nach Anspruch 1 und 4 oder 5, **dadurch gekennzeichnet, daß** die Rastmittel (4) in Form von in den Tracheostomaschenkel (3) eingebrachten Nuten ausgebildet sind.

7. Tracheostomaplatzhalter nach Anspruch 1 und 4 oder 5, **dadurch gekennzeichnet, daß** die Rastmittel (4) in Form von auf den Tracheostomaschenkel (3) aufgebrachten Wulsten ausgebildet sind.

8. Tracheostomaplatzhalter nach Anspruch 1, **dadurch gekennzeichnet, daß** dem Schild (2) Befestigungselemente (8) zur Aufnahme eines Halte- oder Klebebandes zugeordnet sind.

9. Tracheostomaplatzhalter nach Anspruch 1 oder 8, **dadurch gekennzeichnet, daß** dem Schild (2) zumindest im Außenberandungsbereich eine anatomisch an den Hals angepaßte Wölbung gegeben ist.

10. Tracheostomaplatzhalter nach Anspruch 1, **dadurch gekennzeichnet, daß** dem Schild (2) eine mittige Schildausnehmung gegeben ist, die Ausnehmungen (22) aufweist, die einen Eingriff in mit diesen Ausnehmungen (22) korrespondierend festgelegten Wulstbereichen der Rastmittel (4) ermöglichen.

11. Tracheostomaplatzhalter nach Anspruch 1, **dadurch gekennzeichnet, daß** das Verschlußmittel durch einen Stopfen (6) gebildet ist, der über eine Verlustsicherung (7) mit einem Ring (5) verbunden ist.

12. Tracheostomaplatzhalter nach Anspruch 1 und 11, **dadurch gekennzeichnet, daß** der Stopfen (6), die Verlustsicherung (7) und der Ring (5) als einstückiges Spritzteil ausgebildet sind, wobei der Ring (5) in die Rastmittel (4) des Tracheostomaschenkels (3) eingreifend angeordnet ist.

13. Tracheostomaplatzhalter nach Anspruch 11 oder 12, **dadurch gekennzeichnet, daß** der Stopfen (6) mit einer stempelartigen Verlängerung (9) versehen ist.

14. Tracheostomaplatzhalter nach Anspruch 13, **dadurch gekennzeichnet**, der stempelartigen Verlängerung (9) über ihren wesentlichen Längsbereich ein kleinerer Außendurchmesser als der Innendurchmesser des Tracheostomaschenkels (3) gegeben ist und sie nur im Bereich der Austrittsöffnung im Fußanbindungsgebiet des Trachealschenkels (1) dichtend anliegend verdickt ausgeführt ist.

15. Tracheostomaplatzhalter nach Anspruch 1, **dadurch gekennzeichnet, daß** der Tracheostomaschenkel (3) an dem, dem Trachealschenkel (1) abseitigen Ende mit einem Universalkonnektor für eine assistierte Beatmung versehbar ist.

16. Tracheostomaplatzhalter nach Anspruch 1, **dadurch gekennzeichnet, daß** der längere Überhangbereich (1a) mehr ausgedünnte Trachealschenkelabschnitte (11) zugeordnet sind als dem kürzeren Überhangbereich (1b).

## Claims

1. Tracheostoma placeholder for sealing a temporary trachea incision and forming a reliable access possibility to the trachea, said device consisting of a tube-like tracheostoma flange (3) terminating on one side into a tracheal flange (1) designed as an arch in a first dimension (y), whereby the tracheostoma flange (3) and the tracheal flange (1) are a one-part unit and the tracheal flange (1) is in a non-central position to the tracheostoma flange axis (X-X) in a dimension (z) perpendicular to the first dimension so that it shows differently long overhang portions (1a, 1b), whereby the tracheostoma flange (3) can be provided with a closing element at the end facing away from the tracheal flange (1), **characterized in that** the overhang portions (1a, 1b) are provided with several reduced tracheal flange sections (11), and the tracheostoma flange (3) has snap-in means (4) which guarantee the receipt and fixture of a shield (2) at a variably adaptable distance (a) to the contact surface of the tracheal flange (1) to the trachea (T).

2. Tracheostoma placeholder according to claim 1 wherein the tracheal flange (1) has an atraumatic and ellipse-like arch design parallel to the great semi-axis, whereby this arch is adapted to the Trachea (T).

3. Tracheostoma placeholder according to claim 1, wherein the length of the longer overhang portion (1a) has a defined value of the 1.5 fold of the length of the shorter overhang portion (1b).

4. Tracheostoma placeholder according to claim 1, wherein the elements tracheal flange (1), tracheostoma flange (3) and snap-in means (4) are manufactured as a one-piece molded part of an elastic medical material.

5. Tracheostoma placeholder according to claim 4, wherein the elements tracheal flange (1), tracheostoma flange (3) and snap-in means (4) are made of a silicone.

6. Tracheostoma placeholder according to claims 1 and 4 or claim 5, wherein the snap-in means (4) are formed as grooves in the tracheostoma flange (3).

7. Tracheostoma placeholder according to claims 1 and 4 or claim 5, wherein the snap-in means (4) are formed as reinforcements that are applied onto the tracheostoma flange (3).

8. Tracheostoma placeholder according to claim 1, wherein the shield (2) is provided with fixing elements (8) for receiving a supporting or adhesive tape.

9. Tracheostoma placeholder according to claim 1 or 8, wherein the shield (2) is provided with a curvature at least at its outer edge area, whereby said curvature is anatomically adjusted to the neck.

10. Tracheostoma placeholder according to claim 1, wherein the shield (2) is provided with a central opening having recesses (22) which allow the engagement of the shield (2) into the reinforcement areas of the snap-in means (4) corresponding to these recesses (22).

11. Tracheostoma placeholder according to claim 1, wherein the closing element is a plug (6) that is connected with a ring (5) via a loss-preventing unit (7).

12. Tracheostoma placeholder according to claims 1 and 11, wherein the plug (6), the loss-preventing unit (7) and the ring (5) are a one-piece molded component, whereby the ring (5) is positioned in such a way that it engages into the snap-in means (4) of the tracheostoma flange (3).

13. Tracheostoma placeholder according to claim 11 or 12, wherein the plug (6) is provided with a piston-like elongation piece (9).

14. Tracheostoma placeholder according to claim 13, wherein the piston-like elongation piece (9) has an outer diameter along most of its length which is smaller than the inner diameter of the tracheostoma flange (3) and only at the outlet opening of the bottom connection area of the tracheal flange (1) it has a little bit thicker design to ensure the close contact with the flange.

15. Tracheostoma placeholder according to claim 1, wherein the end of the tracheostoma flange (3) facing away from the tracheal flange (1) can be provided with a universal connector for assisting artificial aspiration.

16. Tracheostoma placeholder according to claim 1, wherein the longer overhang portion (1a) is provided with a higher number of reduced tracheal flange sections (11) than the shorter overhang portion (1b).

## Revendications

1. Dispositif d'accès pour trachéotomie faisant fonction d'écarteur, destiné à assurer l'occlusion étanche d'une incision temporaire de la trachée et à permettre simultanément un accès sécurisé à la trachée, composé d'une branche tubulaire (3) du trachéostome dont une des extrémités se transforme en la branche trachéale (1) conçue d'une seule pièce dont la première dimension (y) est courbée et la dimension (z) placée en position perpendiculaire par rapport à la première dimension de la branche trachéale (1), laquelle est disposée de manière non centrale par rapport à l'axe (X-X) de la branche tubulaire du trachéostome (3), de manière à ce que les parties saillantes (1a, 1b,) de la branche trachéale la dépassant, présentent, chacune, des longueurs différentes; l'extrémité de la branche tubulaire du trachéostoma (3) étant opposée à la branche trachéale (1) peut être munie d'un dispositif de fermeture, **caractérisé en ce que** les parties saillantes (1a, 1b) sont dotées de sections trachéales rétrécies (11) et que la branche tubulaire du trachéostome (3) est pourvue de moyens de fixation (4) permettant le logement et la fixation d'un bouclier (2) dans une distance variable (a) par rapport à la face d'application de la branche trachéale (1) entrant en contact avec la trachée (T).

2. Le dispositif d'accès pour trachéotomie faisant fonction d'écarteur, suivant la revendication 1 est **caractérisé en ce que** la branche trachéale (1) conçue prend la forme d'une courbe ellipsoïdale orientée en direction du grand demi-axe et adaptée à la trachée (T) pour éviter tout risque traumatique.

3. Le dispositif d'accès pour trachéotomie faisant fonction d'écarteur, suivant la revendication 1 est **caractérisé en ce que** la dimension de la partie saillante la plus longue (1a) est définie d'une valeur égale à 1,5 fois la longueur de la partie saillante la plus courte (1b).

4. Le dispositif d'accès pour trachéotomie faisant fonction d'écarteur suivant la revendication 1 est **caractérisé en ce que** les composants branche trachéale (1), branche tubulaire du trachéostome (3) et moyens de fixation (4) sont réalisés en une seule pièce moulée par injection dans un matériau médical souple.

5. Le dispositif d'accès pour trachéotomie faisant fonction d'écarteur, suivant la revendication 4 est **caractérisé en ce que** les composants branche trachéale (1), branche tubulaire du trachéostome (3) et moyens de fixation (4) sont en silicone.

6. Le dispositif d'accès pour trachéotomie faisant fonction d'écarteur, suivant les revendications 1 et 4 ou 5 est **caractérisé en ce que** les moyens de fixation (4) se présentent sous forme de rainures appliquées sur la branche tubulaire du trachéostome (3).

7. Le dispositif d'accès pour trachéotomie faisant fonction d'écarteur, suivant les revendications 1 et 4 ou 5 est **caractérisé en ce que** les moyens de fixation (4) prennent la forme de cordons appliqués sur la branche tubulaire du trachéostome (3).

8. Le dispositif d'accès pour trachéotomie faisant fonction d'écarteur, suivant la revendication 1 est **caractérisé en ce qu'**au bouclier (2) sont attribués des éléments de fixation (8) permettant l'attachement d'un ruban de support ou d'un ruban adhésif.

9. Le dispositif d'accès pour trachéotomie faisant fonction d'écarteur suivant les revendications 1 ou 8 est **caractérisé en ce qu'**au moins la surface extérieure du bouclier (2) affecte une forme courbée s'adaptant à l'anatomie du cou.

10. Le dispositif d'accès pour trachéotomie faisant fonction d'écarteur suivant la revendication 1 est **caractérisé en ce que** sur milieu du bouclier (2) se trouve un creux présentant des évidements (22) permettant de relier les cordons correspondants aux moyens de fixation (4).

11. Le dispositif d'accès pour trachéotomie faisant fonction d'écarteur, suivant la revendication 1 est **caractérisé en ce que** l'occlusion est assurée par un bouchon (6) relié à une bague (5) par un élément de fixation (7) empêchant qu'il ne se perde.

12. Le dispositif d'accès pour trachéotomie faisant fonction d'écarteur, suivant les revendications 1 et 11 est **caractérisé en ce que** le bouchon (6), l'élément de fixation (7) l'empêchant de se perdre et la bague elle-même sont exécutés en une seule pièce moulée par injection, la bague (5) étant disposée de sorte qu'elle s'accroche aux moyens de fixation (4) de la branche tubulaire du trachéostome (3).

13. Le dispositif d'accès pour trachéotomie faisant fonction d'écarteur, suivant les revendications 11 ou 12 est **caractérisé en ce que** le bouchon (6) est doté d'une prolongation (9) en forme de tampon.

14. Le dispositif d'accès pour trachéotomie faisant fonction d'écarteur, suivant la revendication 13 est **caractérisé en ce que** sur presque toute la longueur de la prolongation (9) en forme de tampon le diamètre extérieur reste plus étroit que le diamètre intérieur de la branche tubulaire du trachestome (3) et que ce n'est qu' à l'orifice de sortie, dans la zone de contact avec la branche trachéale (1), que cette prolongation devient plus large pour assurer l'étanchéité.

15. Le dispositif d'accès pour trachéotomie faisant fonction d'écarteur, suivant la revendication 1 est **caractérisé en ce que** la branche tubulaire du trachéostome (3) est conçue de sorte qu'à son extrémité opposée à la branche trachéale (1), un adaptateur universel permette d'y coupler un système de respiration assistée.

16. Le dispositif d'accès pour trachéotomie faisant fonction d'écarteur, suivant la revendication 1 est **caractérisé en ce que** les sections rétrécies de la branche trachéale (11) attribuées à la partie saillante la plus longue (1a) sont plus nombreuses que celles attribuées à la partie saillante la plus courte (1b).
